Europäisches Patentamt

European Patent Office

Office européen des brevets

(11) Veröffentlichungsnummer: **0 242 799 B1**

# EUROPÄISCHE PATENTSCHRIFT

(45) Veröffentlichungstag der Patentschrift: **09.12.92**

(21) Anmeldenummer: **87105630.5**

(22) Anmeldetag: **15.04.87**

(51) Int. Cl.5: **C07K 1/00**, C07K 5/02, C07K 7/00, G01N 33/574, //G01N33/534

(54) **Cysteinreiche, gamma-Glutaminsäure und beta-Alanin-Einheiten aufweisende Peptide, Verfahren zu deren Herstellung und ihre Verwendung.**

(30) Priorität: **23.04.86 DE 3613758**

(43) Veröffentlichungstag der Anmeldung: **28.10.87 Patentblatt 87/44**

(45) Bekanntmachung des Hinweises auf die Patenterteilung: **09.12.92 Patentblatt 92/50**

(84) Benannte Vertragsstaaten: **AT BE CH DE FR GB IT LI NL**

(56) Entgegenhaltungen: **EP-A- 0 193 882**

**FEBS, Band 205, Nr. 1, September 1986, Seiten 47-50, Elsevier Science Publishers B.V.; E. GRILL et al.: "Homo-phytochelatins are heavy metal-binding peptides of homo-glutathione containing Fabales"**

(73) Patentinhaber: **INDENA S.p.A.**
**Via Ripamonti, 99**
**I-20141 Milano(IT)**

(72) Erfinder: **Grill, Erwin, Dipl.-Biologe**
**Saumweberstrasse 14**
**W-8000 München 60(DE)**
Erfinder: **Winnacker, Ernst-Ludwig, Prof., Dr., Dipl.-Chem.**
**Dall'Armistrasse 41**
**W-8000 München 19(DE)**
Erfinder: **Zenk, Meinhart H., Prof., Dr., Dipl.-Biologe**
**Pfeivestlstrasse 17**
**W-8000 München 60(DE)**

(74) Vertreter: **Bianchetti, Giuseppe**
**Studio Consulenza Brevettuale Via Rossini, 8**
**I-20122 Milan(IT)**

## Beschreibung

Die Erfindung betrifft cysteinreiche, γ-Glutamin-Säure- und β-Alanin-Einheiten aufweisende Peptide.

Cysteinreiche Proteine tierischen Ursprungs (Metaliothioneine) sind bereits bekannt geworden. Die Aminosäuren dieser Proteine sind durch α-Peptidbindung miteinander verknüpft. Diese Metallothioneine können erhebliche Mengen an Schwermetall binden und dienen so vermutlich im entsprechend belasteten Organismus zur Entgiftung.

(Hierzu Kägi J. Biochem. 89, 1839, (1981)).

Weiterhin sind auch bereits gemäß Agric. Biol. Chem. 49, 71, (1985), aus Spalthefe gewonnene Peptide der Formel

$$H-\gamma\text{-}Glu\text{-}Cys\text{-}\gamma\text{-}Glu\text{-}Cys\text{-}Gly\text{-}OH$$

$$H-\gamma\text{-}Glu\text{-}Cys\text{-}\gamma\text{-}Glu\text{-}Cys\text{-}\gamma\text{-}Glu\text{-}Cys\text{-}Gly\text{-}OH$$

bekannt geworden, sowie Phytochelatine, die aus höheren Pflanzen und Pilzen gewonnen werden können, die durch folgende Aminosäuresequenz beschrieben sind (γ-Glu-Cys)$_n$-Gly, wobei n eine ganze Zahl von 4-7 ist, gemäß Science 230, 674 (1985) und FEBS Letters 197, 115 (1986).

Gegenstand der Erfindung sind Peptide, im folgenden auch als Homo-Phytochelatine bezeichnet, die durch die folgende Aminosäuresequenz charakterisiert sind:

(γ-Glu-Cys)$_n$-β-Ala

wobei n eine ganze Zahl von 2-11 ist.

γ-Glu steht für γ-Glutaminsäure, Cys steht für Cystein,

β-Ala steht für β-Alanin.

Die erfindungsgemäßen Peptide können durch die folgenden Strukturformeln dargestellt werden:

n = 2 γ-Glu-Cys-γ-Glu-Cys-β-Ala

n = 3 γ-Glu-Cys-γ-Glu-Cys-γ-Glu-Cys-β-Ala

n = 4 γ-Glu-Cys-γ-Glu-Cys-γ-Glu-Cys-γ-Glu-Cys-β-Ala

n = 5 γ-Glu-Cys-γ-Glu-Cys-γ-Glu-Cys-γ-Glu-Cys-γ-Glu-Cys-β-Ala

n = 6 γ-Glu-Cys-γ-Glu-Cys-γ-Glu-Cys-γ-Glu-Cys-γ-Glu-Cys-
γ-Glu-Cys-β-Ala

n = 7 γ-Glu-Cys-γ-Glu-Cys-γ-Glu-Cys-γ-Glu-Cys-γ-Glu-Cys-
γ-Glu-Cys-γ-Glu-Cys-β-Ala

n = 8 γ-Glu-Cys-γ-Glu-Cys-γ-Glu-Cys-γ-Glu-Cys-γ-Glu-Cys-
γ-Glu-Cys-γ-Glu-Cys-γ-Glu-Cys-β-Ala

n = 9 γ-Glu-Cys-γ-Glu-Cys-γ-Glu-Cys-γ-Glu-Cys-γ-Glu-Cys-
γ-Glu-Cys-γ-Glu-Cys-γ-Glu-Cys-γ-Glu-Cys-β-Ala

n =10 γ-Glu-Cys-γ-Glu-Cys-γ-Glu-Cys-γ-Glu-Cys-γ-Glu-Cys-
γ-Glu-Cys-γ-Glu-Cys-γ-Glu-Cys-γ-Glu-Cys-γ-Glu-Cys-β-Ala

n =11 γ-Glu-Cys-γ-Glu-Cys-γ-Glu-Cys-γ-Glu-Cys-γ-Glu-Cys-
γ-Glu-Cys-γ-Glu-Cys-γ-Glu-Cys-γ-Glu-Cys-γ-Glu-Cys-
γ-Glu-Cys-β-Ala.

Gegenstand der Erfindung sind ferner Homo-Phytochelatine mit einem Gehalt an Metallkationen. Grundsätzlich kommen alle Metallkationen in Betracht, die mit den Thiolatgruppen des entsprechenden Homo-Phytochelatins eine koordinative Bindung eingehen können. Beispiele hierfür sind insbesondere die Kationen der Metalle Blei, Zinn, Wismut, Titan, Vanadin, Molybdän, Mangan, Kobalt, Nickel, Eisen, Kupfer, Silber, Gold, Platin, Palladium, Zink, Kadmium, Quecksilber, Uran, Arsen, Selen, Technetium, Gallium.

Der Metallgehalt ist naturgemäß abhängig von der Wertigkeit des koordinativ an die Thiolatgruppen gebundenen Kations. Er beträgt 1 bis 4 Mol, bei zweiwertigen Kationen normalerweise 2 Mol, pro Mol Homo-Phytochelatin.

Die erfindungsgemäßen Homo-Phytochelatine sind erhältlich durch Extraktion pflanzlichen Materials. Als pflanzliches Material kommen Arten in Betracht, die Homo-Glutathion enthalten, insbesondere Arten der Überordnung Fabales der höheren Pflanzen.

Beispielhaft genannt sei: Pflanzenmaterial aus der Überordnung der Fabales, insbesondere

Phasaeolus vulgaris mit seinen Zuchtformen

Phasaeolus coccineus

Phasaeolus aureus

Phasaeolus lunatus

Phasaeolus multiflorus

Glycine max (Sojabohne)

Erythrina crista-galli

sowie

Ononis atrix

Lotus ornithopodiodes

Trigonella coerulea

Medicago sativa

Melilotus alba

Trifolium pratense

Lathyrus ochrus

Cicer avietinum

Lens culinaris

Astragalus linsitanicus

Galega officinalis.

Als pflanzliches Material werden vorzugsweise Zellkulturen der genannten Pflanzen eingesetzt. Es können jedoch auch die differenzierten Pflanzen oder Pflanzenteile zur Gewinnung der erfindungsgemäßen Peptide herangezogen werden.

Die Bildung der Homo-Phytochelatine in der Pflanze wird durch Behandeln der Pflanzen mit Metall bzw. mit Metallsalzen induziert. Diese Behandlung kann in jeder geeigneten Weise erfolgen, in der die Pflanzen die Metalle bzw. die Metallkationen aufnehmen können. Vorzugsweise erfolgt die Induzierung der Homo-Phytochelatine durch Behandeln von Zellkulturen mit Metallsalzlösungen im wäßrigen System. Üblicherweise wird dabei so vorgegangen, daß das Zellmaterial in der Metallsalzlösung kultiviert wird, wobei Metallkonzentrationen eingehalten werden, die unterhalb der akuten Phytotoxizität liegen. Die Untergrenze der Metallkonzentrationen liegt bei 1 μmol pro Liter. Die Obergrenze hängt, wie bereits erwähnt, stark von der Phytotoxizität des entsprechenden Metallkations ab. Sie kann bis zu 100 mmol pro Liter betragen. Bevorzugt ist der Bereich von 10 μmol pro Liter bis 1 mmol pro Liter.

Vorzugsweise wird deshalb solches pflanzliches Material zur Gewinnung der Homo-Phytochelatine eingesetzt, das, wie vorstehend beschrieben, in Gegenwart von Metallen bzw. Metallkationen und Metallanionen kultiviert wurde.

Die Extraktion des pflanzlichen Materials erfolgt nach Methoden, nach denen auch bereits bisher Peptide aus Pflanzen extrahiert werden konnten. Üblicherweise wird so vorgegangen, daß die Zellkultur der Pflanzen oder der Zellkulturen zerstört wird. Hierzu wird das pflanzliche Material zweckmäßigerweise eingefroren, z.B. mit flüssigem Stickstoff ggf. zerkleinert und schließlich in einer wäßrigen, leicht alkalischen Zubereitung aufgenommen. Zweckmäßigerweise beträgt der pH-Wert der wäßrigen Zubereitung 7.5 - 9.5. Es können jedoch auch saure Zubereitungen verwendet werden von pH 1 - 3. Oftmals werden als wäßrige Zubereitungen für die genannten pH-Bereiche geeignete Pufferlösungen verwendet. Ggf. nach Abtrennen nicht aufgeschlossenen Pflanzenmaterials erfolgt die Aufarbeitung des Extrakts. Zweckmäßigerweise wird der Extrakt zunächst eingeengt. Dies kann beispielsweise durch Gefriertrocknen oder durch Absorption der Peptide an einen Ionenaustauscher und anschließendes Eluieren bewerkstelligt werden. Danach werden die mitextrahierten Proteine durch Zusatz von Elektrolyt (z.B. Ammoniumsulfat-Lösung) gefällt. Die erfindungsgemäßen Peptide verbleiben dabei in Lösung. Schließlich können weitere Reinigungsschritte erfolgen, wie beispielsweise Ultrafiltration, Gelfiltration oder Fällung der Homo-Phytochelatine mit Kupfersulfat-Lösung. Die Auswahl der Eluate kann jeweils anhand von UV-Messungen nach Maßgabe der typischen Absorptionsbande für die Metall-Thiolatbindung sowie ggf. durch qualitativen Nachweis der SH-Funktionen (beispielsweise mit Ellman's Reagenz) erfolgen.

Sofern das pflanzliche Material im basischen Medium aufgenommen wurde, fallen die Phytochelatine mit einem entsprechenden Gehalt an Metallionen an. Die freien Peptide können beispielsweise durch Ansäuern und Fällung (z.B. $H_2S$-Fällung) der Metallkationen erhalten werden. Alternativ können die mit Metallkationen beladenen Peptide beispielsweise auch mit Hilfe von Komplexbildnern (z.B. Ethylendiamin tetraacetat) vom Metallgehalt befreit werden.

Wird das Pflanzenmaterial in sauren Zubereitungen aufgenommen, fallen die Peptide ohne Metallkationen-Gehalt an.

Die erfindungsgemäßen Peptide der Formel

($\gamma$-Glu-Cys)$_n$ $\beta$-Ala

fallen in der Regel als Gemische an, wobei das Peptid

($\gamma$-Glu-Cys)$_2$ $\beta$-Ala

unter den erfindungsgemäßen Peptiden meist die Hauptkomponente ist. Ihr Anteil beträgt zumeist das 2 - 8-fache der höherkettigen Peptide.

Die typische Häufigkeitsverteilung der erfindungsgemäßen Peptide im Peptidgemisch, bezogen auf die Gesamtmenge erfindungsgemäßer Peptide, ergibt sich aus den folgenden Angaben:

40 - 60 mol% ($\gamma$-Glu-Cys)$_2$ $\beta$-Ala

20 - 40 mol% ($\gamma$-Glu-Cys)$_3$ $\beta$-Ala

10 - 20 mol% ($\gamma$-Glu-Cys)$_4$ $\beta$-Ala

2 - 10 mol% ($\gamma$-Glu-Cys)$_5$ $\beta$-Ala.

Die Glieder mit n = 6 - 11 sind zumeist nur in 1 - 0.001 mol% vertreten.

Falls erwünscht, können die Peptide nach an sich bekannten Methoden der Peptid-Auftrennung getrennt werden. Beispiele hierfür sind die HPL-Chromatographie, die Gelfiltration, Chromatographie an Ionenaustauschern u.a..

Die erfindungsgemäßen Peptide können einzeln oder im Gemisch, in Substanz oder in Form von Zubereitung zur Anwendung kommen, die auch bereits bisher für die Formulierung von Peptid-Zusammensetzungen verwendet werden konnten. Beispielsweise können die erfindungsgemäßen Peptide in an sich bekannter Weise an Trägermaterialien physisorbiert oder chemisch adsorbiert werden. Beispiele für Trägermaterialien sind Alginate, funktionelle Gruppen (wie z.B. Oxirangruppe) aufweisende Agarosen, Zellulose, Polyacrylharze, Gläser, Silikate u.a..

Die erfindungsgemäßen Peptide finden Verwendung in Form von pharmazeutischen Zubereitungen zur Behandlung akuter und chronischer Schwermetallvergiftungen. Andererseits können auch mit Metallkationen beladene erfindungsgemäße Peptide zur Behandlung von Metallmangelerscheinungen bzw. zur Aufrechterhaltung der Homeostasis der entsprechenden Metalle (z.B. Eisen, Zink, Kupfer) verwendet werden.

Weitere Anwendungen ergeben sich auf dem Sektor des Umweltschutzes, beispielsweise zur Klärung von schwermetall-belasteten Abwässern.

Ferner können die erfindungsgemäßen Peptide zur Anreicherung wertvoller Metalle aus wäßrigen Lösungen eingesetzt werden.

Weiterhin können schwermetall-beladene, erfindungsgemäße Peptide zur Gewinnung von entsprechenden Antikörpern eingesetzt werden, die ihrerseits wiederum als Diagnostikum für den Belastungsgrad an Schwermetallen bei Pflanzen dienen.

Außerdem können die mit radioaktivem Metall beladenen Homo-Phytochelatine, an Antikörper gekoppelt, als Diagnostika verwendet werden (z.B. Tumordiagnose).

Die Erfindung wird nun anhand von Beispielen näher erläutert:

Beispiel 1:

1.000 g (entsprechend 54 g Trockengewicht) einer Zellkultur von Glycine max wurden in 4 l einer 100 $\mu$molaren wäßrigen Cadmiumsulfat-Lösung 3 Tage unter ständigem Schütteln kultiviert. Danach wurde die Zellkultur geerntet, mit destilliertem Wasser gewaschen und in flüssigem Stickstoff eingefroren. Die derart aufgeschlossene Zellkultur wurde anschließend in 500 ml Pufferlösung (10 mmolare wäßrige Lösung von Trihydroxymethylaminomethan/HCl/2-Mercaptoethanol) vom pH = 8.6 suspendiert.

Die Suspension wurde zentrifugiert. Der Überstand wurde erneut auf den pH von 8.6 gebracht und soweit verdünnt, daß die Leitfähigkeit < 1 kS betrug. Danach wurde der Überstand über eine Anionenaustauschersäule (Diethylaminoethyl-Agarose-Säule, Bio-Rad, Richmond, USA) geschickt.

Anschließend wurde mit einer wäßrigen Lösung (0,5 mol/l NaCl, 10 mmol/l Trihydroxymethylaminomethan/HCl, pH = 8.6) eluiert. Das Eluat wurde zur Proteinfällung bis zur 85%igen Sättigung mit festem Ammoniumsulfat versetzt. Danach wurde zentrifugiert und der Überstand durch Ultrafiltration (Membran YM-2 der Firma Amicon Davers, GB) von Ammoniumsulfat befreit und auf 15 ml eingeengt.

Schließlich wurde die eingeengte Lösung einer Gelfiltration (Sephadex G-50, Firma Pharmacia, Uppsala, Schweden) bei pH = 7 unterworfen. Der pH-Wert wurde mit Ammoniumacetat entsprechend eingestellt. Durch Eluieren mit 5 mmolarer Ammoniumacetat-Lösung wurden zunächst ein kleiner Anteil hochmolekularen Proteins und anschließend die gewünschten Peptide erhalten.

Nach Lyophilisieren des Eluats wurden 180 mg an Peptidgemisch erhalten. Der Cadmiumgehalt betrug 13.8 Gew.-%.

Beispiel 2:

100 mg des gemäß Beispiel 1 erhaltenen Homo-Phytochelatingemisches wurden in 10 ml 0.01 normaler Salzsäure gelöst. In diese Lösung wurde gasförmiger Schwefelwasserstoff unter Fällung von Cadmiumsulfid eingeleitet. Danach wurde abzentrifugiert und der Überstand lyophilisiert. Es fielen 82 mg an metallfreiem Peptidgemisch an.

Durch HPL-Chromatographie an einer "reversed phase-Säule" (Nucleosil C - 18 der Firma Macherey und Nagel, Düren, BRD) wurde das Peptidgemisch aufgetrennt. Es hatte folgende Zusammensetzung:

46.3 mol% $(\gamma\text{-Glu-Cys})_2$ $\beta$-Ala

37.8 mol% $(\gamma\text{-Glu-Cys})_3$ $\beta$-Ala

11.7 mol% $(\gamma\text{-Glu-Cys})_4$ $\beta$-Ala

3.6 mol% $(\gamma\text{-Glu-Cys})_5$ $\beta$-Ala

0.3 mol% $(\gamma\text{-Glu-Cys})_6$ $\beta$-Ala

0.3 mol% $(\gamma\text{-Glu-Cys})_{7-11}$ $\beta$-Ala.

Beispiel 3:

50 mg des metallfreien Peptidgemisches (gemäß Beispiel 2) wurden nach Reduktion mit Natriumborhydrid in 10 ml 0.01 normaler Salzsäure aufgenommen und mit 250 $\mu$mol Eisensulfat in Form einer 1 molaren Lösung versetzt. Es wurde unter Stickstoff-Atmosphäre gearbeitet. Danach wurde mit 0.01 normaler Natronlauge neutralisiert (pH = 7.5). Die so erhaltene Lösung wurde schließlich einer Agarose-Säule (Sephadex G-25, Pharmacia, Uppsala, Schweden) aufgegeben. Nach Eluieren mit Wasser und Lyophilisieren des Eluats wurde mit Eisen beladenes Peptidgemisch in einer Ausbeute von 51 mg erhalten.

**Patentansprüche**

1. Peptide, gekennzeichnet durch die folgende Aminosäursequenz:
   $(\gamma\text{-Glu-Cys})_n$ $\beta$-Ala
   wobei n eine ganze Zahl von 2 - 11 ist.

2. Peptid nach Anspruch 1, gekennzeichnet durch einen Gehalt an Metallkationen von 1 bis 4 mol, pro mol Peptid.

3. Verfahren zur Herstellung eines Peptids nach Anspruch 2, dadurch gekennzeichnet, daß
   a) Pflanzenmaterial aus der Überordnung Fabales unter Einwirkung von Metallsalzen kultiviert wird und
   b) das gemäß a) gewonnene Pflanzenmaterial extrahiert wird.

4. Verfahren zur Herstellung des Peptids nach Anspruch 1, dadurch gekennzeichnet, daß ein Peptid nach Anspruch 2 durch Fällungs- und/oder Komplexbildungsreaktion von seinem Metallgehalt befreit wird.

5. Verwendung des Peptids gemäß Anspruch 1 zur Herstellung einer pharmazeutischen Zubereitung zur Entfernung von Schwermetallen aus schwermetall-belasteten physiologischen Systemen.

6. Verwendung des Peptids gemäß Anspruch 2 zur Herstellung einer pharmazeutischen Zubereitung zur Aufrechterhaltung der Homeostasis in physiologischen Systemen.

7. Verwendung des Peptids gemäß Anspruch 2 zur in vitro Tumordiagnose.

**Claims**

5

1. Peptides characterized by the following amino acid sequences:
   ($\gamma$-Glu-Cys)$_n$ $\beta$-Ala $\underline{n}$ being a whole number from 2 - 11.

2. A peptide as in Claim 1, characterized by a content of metal cations of from 1 to 4 mol per mol of peptide.

3. A method of production of a peptide as in Claim 2, characterized in that
   (a) vegetable material is cultivated from the higher order of Fabales under the influence of metal salts; and
   (b) the vegetable material produced in accordance with (a) is extracted.

4. A method for preparing a peptide as in Claim 1, characterized in that a peptide as in Claim 2 is freed of its metal content through precipitation reaction and/or complexing reaction.

5. Use of the peptide in accordance with Claim 1 for the production of a pharmaceutical preparation for the removal of heavy metals from physiological systems loaded with heavy metals.

6. Use of the peptide in accordance with Claim 2 for the production of a pharmaceutical preparation for maintenance of homeostasis in physiological systems.

7. Use of the peptide in accordance with Claim 2 for the diagnosis of tumours <u>in vitro</u>.

**Revendications**

1. Peptides caractérisés par la séquence suivante d'acides aminés :
   ($\gamma$ - Glu - Cys)$_n$ $\beta$ - Ala
   où n est un nombre entier de 2 - 11.

2. Peptide selon la revendication 1, caractérisé par une teneur en ions métalliques de 1 à 4 moles, par mole de peptide.

3. Procédé de préparation d'un peptide selon la revendication 2, caractérisé par le fait que :
   (a) du matériel végétal provenant du superordre Fabales est cultivé sous l'action de sels métalliques ; et
   (b) le matériel végétal obtenu selon (a) est soumis à une extraction.

4. Procédé de préparation du peptide selon la revendication 1, caractérisé par le fait qu'un peptide selon la revendication 2 est débarrassé de sa teneur en métaux par une réaction de précipitation et/ou de complexation.

5. Utilisation du peptide selon la revendication 1 pour la fabrication d'une préparation pharmaceutique pour l'élimination des métaux lourds dans des systèmes physiologiques chargés en métaux lourds.

6. Utilisation du peptide selon la revendication 2 pour la fabrication d'une préparation pharmaceutique pour le maintien de l'homéostasie dans des systèmes physiologiques.

7. Utilisation du peptide selon la revendication 2 pour le diagnostic <u>in vitro</u> des tumeurs.